(19) Europäisches Patentamt
European Patent Office
Office européen des brevets

(11) **EP 2 347 791 A1**

## EUROPEAN PATENT APPLICATION
published in accordance with Art. 153(4) EPC

(43) Date of publication:
**27.07.2011 Bulletin 2011/30**

(21) Application number: **10809758.5**

(22) Date of filing: **12.05.2010**

(51) Int Cl.:
*A61N 5/06* (2006.01)     *A61B 1/00* (2006.01)
*A61B 6/03* (2006.01)     *G01T 1/161* (2006.01)

(86) International application number:
**PCT/JP2010/058000**

(87) International publication number:
**WO 2011/021412 (24.02.2011 Gazette 2011/08)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **19.08.2009 JP 2009190288**

(71) Applicant: **OLYMPUS MEDICAL SYSTEMS CORP.
Tokyo 151-0072 (JP)**

(72) Inventor: **TAKEI, Shunji
Tokyo 151-0072 (JP)**

(74) Representative: **Gunzelmann, Rainer
Wuesthoff & Wuesthoff
Patent- und Rechtsanwälte
Schweigerstraße 2
81541 München (DE)**

(54) **MEDICAL SYSTEM AND MEDICAL CONTROL METHOD**

(57)      A PET-CT apparatus 4 arranged outside the body of a patient 2 detects gamma ray radiated from a drug accumulation region 10 of a drug which is dosed to the patient 2 in advance and which accumulates in a cancer tissue, and acquires information of position and accumulation density of the drug accumulation region 10. A capsule medical apparatus 3 inserted into the body of the patient 2 detects the gamma ray, and when an intensity at an estimation value at a distance suitable for treatment is obtained, a determination and control unit 47 causes a light emitting unit of the capsule medical apparatus 3 to emit light, and the light emitting unit thus performs treatment for therapeutic procedure for a cancer tissue.

## FIG.1

EP 2 347 791 A1

**Description**

Technical Field

The present invention relates to a medical system and a medical control method for performing treatment and the like for a therapeutic procedure for a living organism.

Background Art

In a medical field, in some cases, various medical apparatuses such as an X-ray apparatus or an X-ray tomography apparatus (an X-ray CT apparatus) and an endoscope apparatus are used in combination.

For example, Japanese Patent Application Laid-Open Publication No. 05-285098 discloses an apparatus including an endoscope that obtains an observation image of a subject region and an X-ray apparatus that obtains a perspective image of the subject region, the apparatus combining the observation image obtained by the endoscope and the perspective image to display both the images on a monitor.

The apparatus of the conventional example includes a noise reduction circuit. The noise reduction circuit suppresses a noise component due to X-ray radiation when radiation of an X-ray is turned on.

In recent years, a cancer diagnosis new technique using a molecular target drug starts to attract attention. Possibility of application to medical treatment is examined in combinations with various medical apparatuses.

For example, there is a positron emission type tomography apparatus (PET-CT apparatus) that doses a specific drug, which is obtained by adding a substance that generates a positive electron (positron) to a specific drug such as a molecular target drug including a functional group having a characteristic of combining with biological protein that specifically develops in cancer cells, to a living organism and detects a gamma ray generated by recombination of the positive electron and an electron to detect presence and positions of cancer cells in which the specific drug accumulates.

As a conventional example of the PET-CT apparatus, for example, there is Japanese Patent Application Laid-Open Publication No. 2006-304860. In an apparatus of the conventional example, a gantry for PET and a gantry for X-ray CT are arranged side by side such that one bed can be shared. A jack mechanism for lifting and lowering the bed is provided to make it easy to perform maintenance work.

For example, an endoscope apparatus is proposed that has the purpose of performing presence diagnosis for cancer or qualitative diagnosis for malignancy and the like by giving a fluorescent label to a living organism as the molecular target drug and capturing fluorescent light generated when excitation light is radiated from the inside of the living organism.

Further, a drug is also beginning to be developed that contains a photosensitive substance as the molecular target drug and has the purpose of performing a therapeutic procedure simultaneously with diagnosis.

Thus, the greatest merit of the diagnosis technique employing specific drugs such as a molecular target drug is the capability to yield a unique effect through combination with various medical apparatuses.

However, since the medical apparatuses respectively have significantly different functions, it is necessary to properly use the medical apparatuses in accordance with the purpose of examination and therapeutic procedure, and it is expected that dosing a specific drug for each examination is necessitated every time an examination is made.

For example, the above-mentioned PET-CT apparatus can examine the entire body for the presence of an early cancer but cannot directly approach a found lesion region or affected region to sample a living organism tissue, and therefore cannot obtain a pathologic definite diagnosis.

In addition, even when a diagnosis is defined that a therapeutic procedure should be performed on a lesion region using a molecular target drug, such therapeutic procedure cannot be conducted with the PET-CT apparatus alone if it is necessary to directly approach and administer a treatment on the lesion region.

In contrast to this, an endoscopic approach to a found lesion region enables to directly sample a living organism tissue and to perform a therapeutic procedure or a treatment for symptom moderation or the like from within a living organism. However, to make an accurate endoscopic approach to a lesion region found by a PET-CT apparatus, it is required to perform an examination by again dosing a molecular target drug targeted for the same living organism molecule.

In this case, since it takes time until the therapeutic procedure or treatment is conducted, a problem is expected to rise that it becomes difficult to efficiently perform medical actions.

The present invention was made in view of the above and has an objective to provide a medical system and a medical control method capable of efficiently performing a treatment on a living organism or predetermined operations relating to the treatment using plural medical apparatuses having different functions with respect to a living organism.

The present invention has another objective to provide a medical system and a medical control method capable of efficiently performing a treatment or predetermined operations such as a biopsy relating to the treatment, based on plural medical apparatuses having different functions with respect to a living organism and living organism information obtained by using a specific drug.

Disclosure of Invention

Means for Solving the Problem

A detecting apparatus according to an aspect of the present invention includes:

a first medical apparatus including a first detecting unit for acquiring first living organism information from a living organism;
a second medical apparatus including a second detecting unit for acquiring second living organism information from a living organism and an operation unit that performs a predetermined operation on the basis of the first living organism information received from the first medical apparatus; and
a control unit provided in the second medical apparatus, the control unit controlling an operation of the operation unit on the basis of the second living organism information.

A medical control method according to another aspect of the present invention includes:

a first step of detecting, with a first medical apparatus, a radiation emitted from a specific drug that specifically combines with a lesion tissue in a living organism and calculating a position and accumulation density of a drug accumulation region where the specific drug accumulates;
a second step of calculating, using information acquired by the first step, an estimation value of intensity of the radiation detected by a second medical apparatus at a predetermined distance from the drug accumulation region;
a third step of detecting radiation from the drug accumulation region with a second medical apparatus inserted into the living organism;
a fourth step of determining whether an intensity of radiation detected by the second medical apparatus exceeds the estimation value; and
a fifth step of performing control for applying, when a determination result indicates that an intensity of radiation detected by the second medical apparatus exceeds the estimation value, treatment for a therapeutic procedure to the drug accumulation region with the second medical apparatus.

Brief Description of the Drawings

Fig. 1 is a perspective view showing an overall configuration of a medical system according to a first embodiment of the present invention;
Fig. 2 is a diagram showing a configuration of a capsule medical apparatus;
Fig. 3 is a block diagram showing a configuration of a PET-CT apparatus and the like included in the medical system;
Fig. 4 is a diagram showing a positional relation with a gamma ray detecting element that detects a gamma ray from a drug accumulation region of a patient;
Fig. 5 is a diagram showing a positional relation between the drug accumulation region and the capsule medical apparatus inserted into the patient;
Fig. 6 is a flowchart showing an example of a procedure of a medical control method according to the first embodiment;
Fig. 7 is a diagram showing a state in which the capsule medical apparatus reaches near the drug accumulation region;
Fig. 8 is a diagram showing a state in which the capsule medical apparatus causes a light emitting unit to emit light and performs a treatment operation for a therapeutic procedure;
Fig. 9 is a diagram showing a configuration of a capsule medical apparatus in a modification together with a state of an operation of the capsule medical apparatus;
Fig. 10 is a diagram showing a configuration of a capsule medical apparatus in a second embodiment of the present invention together with a state of an operation of the capsule medical apparatus;
Fig. 11 is a diagram showing a configuration of a capsule medical apparatus in a third embodiment of the present invention together with a state of an operation of the capsule medical apparatus;
Fig. 12 is a diagram showing a schematic configuration of a medical system according to a fourth embodiment of the present invention together with a state of an operation of the medical system;
Fig. 13 is a diagram showing a configuration of a part of a medical system according to a fifth embodiment of the present invention together with a state of an operation of the part of the medical system; and
Fig. 14 is a diagram showing a configuration of a part of the medical system according to the fifth embodiment of the present invention together with a state of an operation of the part of the medical system.

Best Mode for Carrying Out the Invention

Embodiments of the present invention are explained below with reference to the drawings.

(First Embodiment)

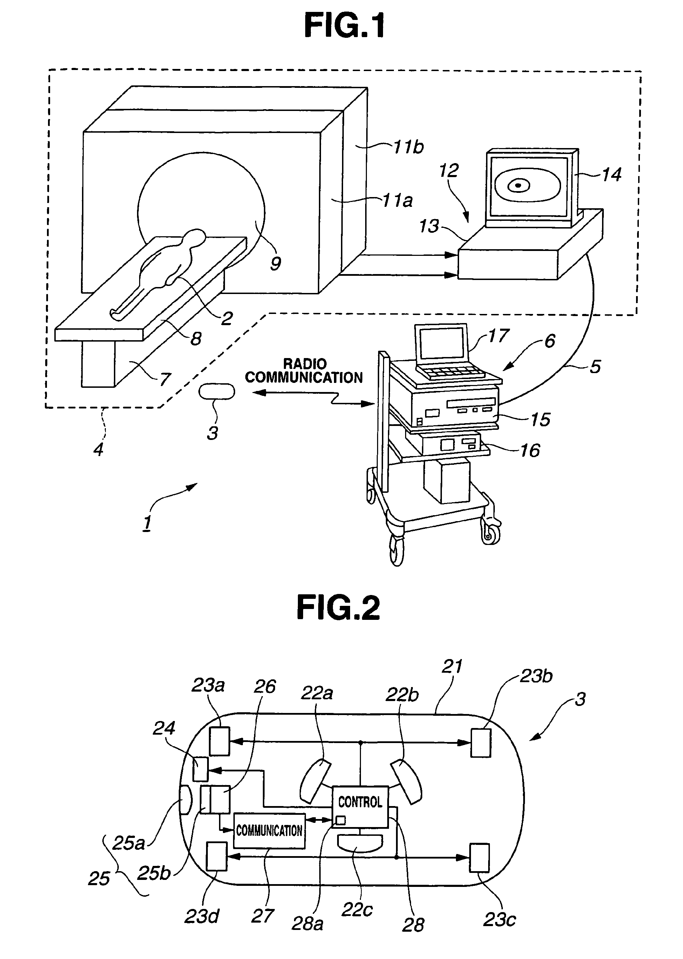

Fig. 1 shows a medical system 1 according to a first embodiment of the present invention. The present embodiment includes an external medical apparatus arranged outside a living organism and including a first detecting unit for acquiring first living organism information from the living organism and an internal medical apparatus arranged inside the living organism and including a second detecting unit for acquiring second living organism information from the living organism. The internal medical apparatus includes an operation unit that performs a predetermined operation on the basis of the first living organism information received from the external medical apparatus and a control unit that controls the operation unit on the basis of the second living organism information. The medical system 1 is specifically explained below.

As shown in Fig. 1, the medical system 1 includes a capsule medical apparatus 3 as an internal medical apparatus that is inserted into or arranged in a body of a patient 2 from a mouth of the patient 2 and performs a predetermined operation.

The medical system 1 includes a PET-CT apparatus (positron emission type tomography apparatus) 4 as the external medical apparatus including a first detecting unit for acquiring, as first living organism information, from an outside of the patient 2, a three-dimensional position (also simply referred to as position) and accumulation density of a drug accumulation area or a drug accumulation region 10 where a molecular target drug as a specific drug dosed to the patient 2 in advance accumulates.

In the present embodiment, for a therapeutic procedure for a living organism tissue of a lesion region (for example, a cancer tissue as a lesion tissue), a molecular target drug having a substance containing a photosensitive substance that specifically combines with, for example, the cancer tissue and a positive electron (positron) as an antimatter of an electron is dosed to the patient 2 in advance.

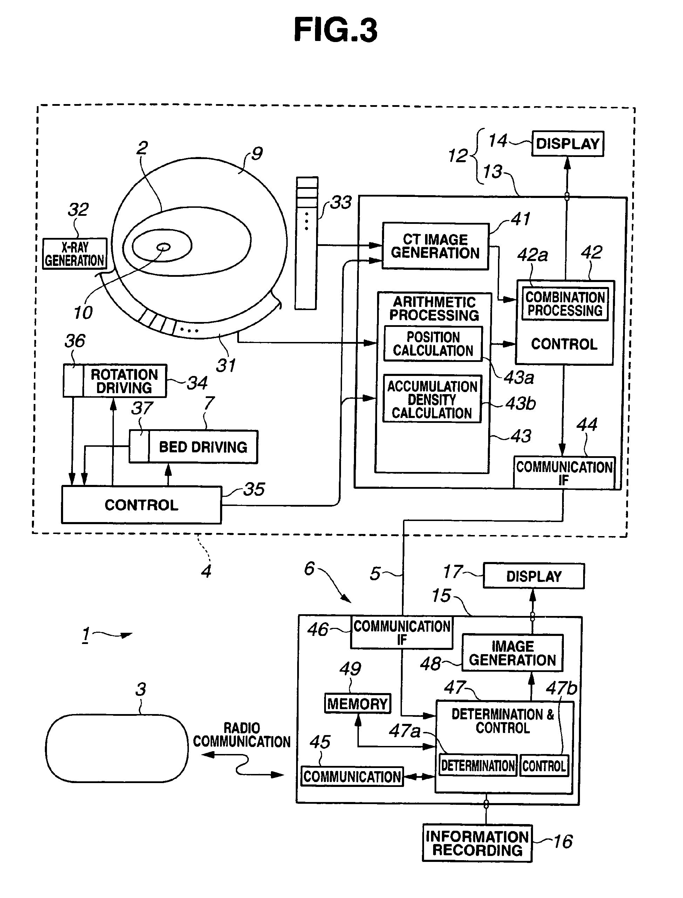

The dosed molecular target drug accumulates in the lesion region where the cancer tissue is present (the lesion region where the molecular target drug accumulates is hereinafter referred to as drug accumulation region 10). A gamma ray is generated when the positive electron recombines with the electron included in the living organism in the drug accumulation region 10. A gamma ray detecting unit 31 (see Fig. 3) in a PET apparatus unit in the PET-CT apparatus 4 as a first detecting unit detects the gamma ray. An arithmetic processing unit 43 (see Fig. 2) in a processing unit main body 13 generates first living organism information.

Since the gamma ray has high permeability to the living organism, the gamma ray can be detected not only in the living organism but also from the outside of the living organism.

The medical system 1 includes the capsule medical apparatus 3 inserted into an inside of the patient 2 and an information processing apparatus 6 that is arranged outside the patient 2, communicates with the capsule medical apparatus 3 by radio, and acquires (receives) information concerning a position and accumulation density of the drug accumulation region 10 as the first living organism information generated by the PET-CT apparatus 4 from the PET-CT apparatus 4 via a communication cable 5.

In Fig. 1, the communication cable 5 for performing communication is shown as an example. However, the information processing apparatus 6 is not limited to a configuration for performing communication by wire and may be a configuration for performing communication by radio (radio wave or light). A USB cable may be adopted as the communication cable 5 (these are generally referred to as communication unit).

Not only the capsule medical apparatus 3 but also a medical apparatus including the capsule medical apparatus 3 and the information processing apparatus 6 can be regarded as a medical apparatus including a second detecting unit and an operation unit arranged in a body.

The PET-CT apparatus 4 includes, together with a bed 8 moved by a bed driving unit 7, a gantry for PET 11a and a gantry for CT 11b in which a circular (cylindrical) gantry opening 9, which can accommodate the patient 2 placed on the bed 8, is provided. In Fig. 1, a configuration in which the two gantries 11a and 11b are arranged adjacent to each other in a longitudinal direction of the bed 8 is shown as an example. However, the configuration may be a configuration including an integrated one gantry.

Respective detection signals (detection information) generated by the gantry for PET 11a and the gantry for CT 11b are outputted to a processing unit 12 via a signal line.

The processing unit 12 performs arithmetic processing for the detection signals with a processing unit main body 13 and generates information concerning a position and accumulation density of the drug accumulation region 10 of the molecular target drug by the gamma ray. The processing unit 12 generates a tomography image (a PET image) of the drug accumulation region 10 and a CT image of an X-ray transmitted through the patient 2, further generates a combined image (a PET-CT image) obtained by combining both the images, and displays the combined image on a display unit 14.

The information processing apparatus 6 to which the information concerning the position and the accumulation density of the drug accumulation region 10 is inputted from the processing unit main body 13 via the communication cable 5 includes an information processing unit 15 mounted on, for example, a cart, an information recording unit 16 that records information processed by the information processing unit 15, and a display unit 17 that displays processed image information and the like.

The information processing unit 15 performs, referring to detection information of a gamma ray in a detection signal from the PET-CT apparatus 4, arithmetic processing for estimating an estimation value Ies of intensity of a gamma ray detected by the capsule medical apparatus 3 in an area where the capsule medical apparatus 3 is at a predetermined distance Ls, which enables a procedure, to the drug accumulation region 10.

It is also possible that the information processing unit 15 does not calculate the estimation value Ies but the processing unit main body 13 calculates the estimation value Ies and sends the estimation value Ies to the information processing unit 15. The information processing unit 15 determines whether the intensity Ide of a gamma ray detected by the capsule medical apparatus 3 satisfies a condition that the intensity is equal to or higher than the estimation value Ies and transmits a result of the determination to the capsule medical apparatus 3. A capsule control unit 28 (see Fig. 2) in the capsule medical apparatus 3 that receives the determination result performs control of a predetermined operation by an operation unit provided in the capsule medical apparatus 3, for example, an operation for radiating therapeutic light. In other words, the capsule control unit 28 controls an operation of the operation unit on the basis of information concerning the estimation value Ies calculated from the first living organism information.

Therefore, the capsule medical apparatus 3 incorporates, in an armor container 21 having a capsule shape as shown in Fig. 2, plural gamma ray sensors 22i (i = a, ..., and e) as second detecting units for acquiring gamma intensity, which is radiated from the drug accumulation region 10, as second living organism information.

A signal level (i.e., intensity) of gamma ray detection signal (detection information) outputted from the gamma ray sensors 22i is the second living organism information. Therefore, the gamma ray sensors 22i play both a function of the second detecting unit for detecting a gamma ray radiated from the drug accumulation region 10 and a function of acquiring or generating the second living organism information as intensity of the gamma ray.

The capsule medical apparatus 3 includes, in the armor container 21, an illumination unit 24, an image pickup unit 25, a signal processing unit 26, a communication unit 27, and a capsule control unit (in the figure, abbreviated as control) 28 that controls the entire apparatus including the operation unit.

The capsule medical apparatus 3 includes, in the armor container 21, as an operation unit that performs a predetermined operation, plural light emitting units 23j (j = a, ..., and d) that generate therapeutic light radiated to an outside of the armor container 21. The capsule control unit 28 has a function of a sensor control unit 28a for controlling operations of the gamma ray sensors 22i, for example, setting of a time interval for detecting a gamma ray by the gamma ray sensors 22i and stopping of gamma ray detection after a procedure by the operation unit.

The communication unit 27 performs two-way radio communication with a communication unit 45 (see Fig. 3) of the information processing apparatus 6. The gamma ray sensors 22i detect a gamma ray as a radiation. A detection signal of the gamma ray is transmitted from the communication unit 27 to the information processing unit 15 by radio via the capsule control unit 28.

The gamma ray sensors 22i may be formed by using, for example, a semiconductor multilayer Compton camera (hereinafter, Compton camera). The Compton camera is an image pickup apparatus that reconfigures a Compton scattering locus of a gamma ray, which occurs in a semiconductor multilayer film, on the basis of kinematics and detects an incident direction and energy of the gamma ray.

As in the present embodiment, when the gamma ray sensors 22i are used, it is possible to reduce size of the gamma ray sensors 22i by limiting functions to a gamma ray detecting function and mount the gamma ray sensors 22i in the capsule medical apparatus 3 without necessity of acquiring two-dimensional image information.

The information processing unit 15 transmits a control signal corresponding to the determination result to the capsule medical apparatus 3 by radio. The capsule control unit 28 in the capsule medical apparatus 3 performs control for causing the light emitting units 23j to emit light. The light emitting units 23j radiate therapeutic light to the outside of the armor container 21.

A photosensitive substance is contained in a molecular target drug. Specifically, active oxygen is generated by the radiation of the therapeutic light. The active oxygen operates (functions) to kill cancer cells of a cancer tissue in which the molecular target drug accumulates.

In this case, a specific drug combining with protein that specifically develops in the cancer cells is used as the molecular target drug. This makes it possible to perform a therapeutic procedure targeting only the cancer cells by causing the active oxygen by the therapeutic light to act on the specific drug combining with the cancer cells.

As shown in Fig. 2, under radiation of illumination light in a visible region by the illumination unit 24, the image pickup unit 25 including an object lens 25a and an image pickup device 25b performs picking up an image of an inside of an illuminated body cavity.

An image pickup signal outputted from the image pickup device 25b including a CCD, a MOS imager, or the like is inputted to the signal processing unit 26. The signal processing unit 26 generates a video signal of an endoscope image from the image pickup signal by performing signal processing. Further, the signal processing unit 26 modulates the video signal and transmits the video signal to the outside of the capsule medical apparatus 3 by radio via the communication unit 27.

The communication unit 27 transmits the video signal of the endoscope image by the image pickup unit 25 and the detection signal of the gamma ray sensors 22i by radio, for example, in a time division manner.

In the present embodiment, the capsule medical apparatus 3 has a function of a capsule endoscope including the illumination unit 24, the image pickup unit 25, and the signal processing unit 26. However, the capsule medical apparatus 3 may have a configuration not including these units.

Fig. 3 shows a schematic configuration of the PET-CT apparatus 4 and the information processing apparatus 6.

As shown in Fig. 3, the PET-CT apparatus 4 includes, as the first detecting unit for acquiring the first living organism information, the gamma ray detecting unit 31 (as a radiation detecting unit) that detects a gamma ray as a radiation radiated, in recombination of a positive electron, from a specific region of a living organism, specifically, the drug accumulation region 10 where cancer cells accumulate. The gamma ray detecting unit 31 is arranged along a circumference of the gantry opening 9.

The gamma ray emitted from the drug accumulation region 10 in the body of the patient 2 as explained above is detected by the gamma ray detecting unit 31 including plural gamma ray detecting elements arrayed in a circumferential direction and a detection signal is outputted to the processing unit main body 13.

In Fig. 3, the gamma ray detecting unit 31 is arranged over an entire circumference of an inner wall surface on which the patient 2 is accommodated in the gantry opening 9. However, the gamma ray detecting unit 31 may be arranged to cover a part of an arc. In that case, the gamma ray detecting unit 31 performs detection of a gamma ray while being driven to rotate.

The gantry for CT 11b includes an X-ray generating unit 32 that is arranged in a predetermined position in the gantry opening 9, generates an X-ray, and radiates the X-ray on the patient 2 accommodated on an inner side of the gantry opening 9 and an X-ray detecting unit 33 including plural X-ray detecting elements arrayed in a line shape or an arc shape that detect the X-ray transmitted through the patient 2. The X-ray detecting unit 33 outputs a detection signal of the X-ray to the processing unit main body 13.

A movable unit including the X-ray generating unit 32 and the X-ray detecting unit 33 in the gantry for CT 11b is driven to rotate around a gantry center axis by a rotation driving unit 34.

Driving operations of the bed driving unit 7 and the rotation driving unit 34 are controlled by a control unit 35 in the PET-CT apparatus 4. The control unit 35 also controls an operation of generation (radiation) of an X-ray by the X-ray generating unit 32.

Rotating positions of the X-ray generating unit 32 and the X-ray detecting unit 33 by the rotation driving unit 34 are detected by a rotating position detecting unit 36 such as a rotary encoder. A detection signal of the rotating positions is inputted to the control unit 35. A detection signal of a position sensor 37 that detects a driving position (a moving position) by the bed driving unit 7 is also inputted to the control unit 35.

The X-ray generating unit 32 generates an X-ray while being driven to rotate. The X-ray detecting unit 33 detects the X-ray transmitted through the patient 2.

A detection signal of the X-ray detecting unit 33 is inputted to a CT image generating unit 41 included in the processing unit main body 13. In this case, a detection signal of a rotating position of the movable unit of the gantry and a detection signal of a driving position by the bed driving unit 7 are also inputted to the CT image generating unit 41 via the control unit 35.

The CT image generating unit 41 generates a CT image corresponding to a structure of an organ, a skeleton, or the like of the patient 2 referring to the detection signal of the rotating position or the like.

The CT image generating unit 41 outputs the generated CT image to a control unit 42 in the processing unit main body 13.

A detection signal of the gamma ray detecting unit 31 is inputted to the arithmetic processing unit 43 in the processing unit main body 13 together with, for example, a position signal of the gamma ray detecting elements.

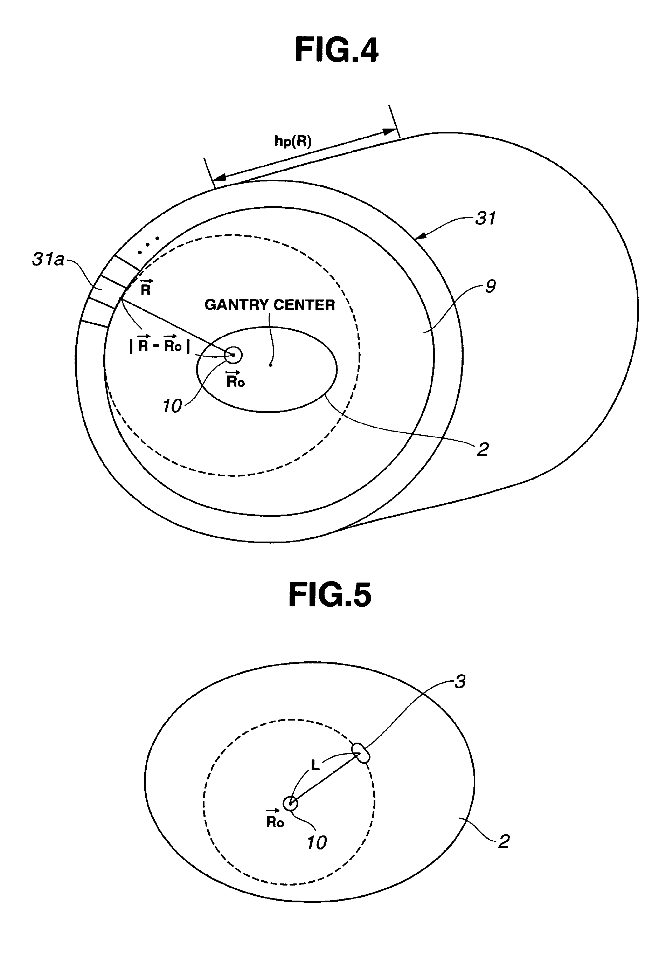

The arithmetic processing unit 43 configures the gamma ray detecting unit 31 as explained later with reference to Fig. 4. The arithmetic processing unit 43 calculates a two-dimensional position of the drug accumulation region 10 and calculates accumulation density of a drug from information concerning intensity of a gamma ray detected according to arrangement positions of gamma ray detecting elements 31a arranged along the circumference of the gantry opening 9.

In this case, it is possible to calculate a three-dimensional position and accumulation density of the drug accumulation region 10 by referring to position information of the position sensor 37.

In this way, the arithmetic processing unit 43 calculates the (three-dimensional) position of the drug accumulation region 10 and an intensity distribution of a gamma ray emitting source, in other words, accumulation density information of the molecular target drug.

Specifically, the arithmetic processing unit 43 has functions of a position calculating unit 43a and an accumulation density calculating unit 43b for the drug accumulation region 10. Accumulation density calculation is explained later with reference to Figs. 4 and 5.

The arithmetic processing unit 43 outputs information concerning both the position and the accumulation density to the control unit 42. The control unit 42 includes a combination processing unit 42a that combines the CT image from the CT image generating unit 41 and the information concerning both the position and the accumulation density from the arithmetic processing unit 43. The arithmetic processing unit 43 displays an image combined by the combination processing unit 42a on the display unit 14.

The control unit 42 transmits the information concerning both the position and the accumulation density to the information processing unit 15 of the information processing apparatus 6 through a communication interface (in the figure, communication IF) 44 and the communication cable 5.

The information processing unit 15 includes a communication unit 45 that performs radio communication with the capsule medical apparatus 3, a communication interface 46 that performs communication with the processing unit main body 13, a determination and control unit 47 that performs determination and control, and an image generating unit 48 that performs image generation processing.

The information concerning both the position and the accumulation density is inputted to the determination and control unit 47 via the communication interface 46. The detection signal of the gamma ray sensors 22i and the video signal of the endoscope image from the capsule medical apparatus 3 are inputted to the determination and control unit 47 via the communication unit 45.

The determination and control unit 47 sequentially records these inputted signals in the information recording unit 16 and temporarily stores, for example, the information concerning both the position and the accumulation density of the drug accumulation region 10 necessary for control in a memory 49.

The determination and control unit 47 estimates, referring to the intensity information of the detection signal of the gamma ray from the drug accumulation region 10 detected by the PET-CT apparatus 4, an estimation value of intensity of a gamma ray detected by the capsule medical apparatus 3 when the capsule medical apparatus 3 is at a specific distance. Specifically, the determination and control unit 47 estimates the estimation value Ies of intensity of a gamma ray detected by the capsule medical apparatus 3 when a distance from the drug accumulation region 10 to the capsule medical apparatus 3 is the predetermined distance Ls suitable for performing an operation for a therapeutic procedure by the capsule medical apparatus 3.

The determination and control unit 47 stores the estimation value Ies in the memory 49. The determination and control unit 47 monitors a detection signal by the gamma ray sensors 22i of the capsule medical apparatus 3. The determination and control unit 47 has a function of a determining unit 47a that performs determination whether the intensity Ide of the gamma ray detected by the capsule medical apparatus 3 satisfies a condition that the intensity Ide is equal to or larger than the estimation value Ies.

$$\text{Ide} \geq \text{Ies} \quad (1)$$

When a determination result indicates that Expression (1) is satisfied, the determination and control unit 47 transmits a control signal to the capsule control unit 28 of the capsule medical apparatus 3 via the communication unit 45. The capsule control unit 28 has a function of a control unit that performs control for causing the operation unit of the capsule medical apparatus 3 to perform a predetermined operation. The determination and control unit 47 has a function of a control unit 47b that performs control of an operation of the information processing apparatus 6.

As a modification of the configuration shown in Fig. 3, for example, the determination and control unit 47 may transmit the estimation value Ies to the capsule control unit 28 of the capsule medical apparatus 3 via the communication unit 45 and the capsule control unit 28 may perform the function of the determination of the determining unit 47a.

In this case, the capsule control unit 28 in the capsule medical apparatus 3 performs the determination of the determining unit 47a and further performs control corresponding to a determination result. The light emitting units 23j as the operation units perform operation for emitting light as a predetermined operation according to the control.

Fig. 4 shows an arrangement relation between the drug accumulation region 10 in the gantry for PET 11a and the gamma ray detecting elements 31a of the gamma ray detecting unit 31.

The number of photons of a gamma ray radiated per one second from a unit volume in the drug accumulation region 10 is represented as indicated by the following Equation (2).

$$N = \rho \times P \times 2 * T \qquad (2)$$

$\rho$: accumulation density of a radioactive isotope (a positive electron)
P: collapse speed (the number of positive electrons radiated per unit density in one second)
T: radiation probability (about 99.7%) of a gamma ray by recombination of a positive electron and an electron
P is calculated according to elapsed time from time of drug generation

A position of the drug accumulation region 10 in the patient 2 arranged in the gantry for PET 11 a is represented as Ro (in Fig. 4, Ro is indicated by an arrow of a vector sign) and positions of the gamma ray detecting elements 31a arranged along the circumference of the circular opening of the gantry for PET 11a are represented as R. The gamma ray detecting elements 31 a are integrated over a gantry entire circumference to calculate the number of photons (corresponding to intensity) Ip of a gamma ray detected by Equation (3).

$$Ip = \int N \times h_p(R)/(\pi * (R-Ro)^2)dR \qquad (3)$$

$h_p(R)$: a distance in a length direction of a gantry cylinder in the gamma ray detecting element in the position R from a gantry center
R-Ro: a distance from the drug accumulation region to the gamma ray detecting element on the circumference of the gantry opening

A total number of photons N is known information from the number of photons $I_p$ calculated by Equation (3). The accumulation density $\rho$ of the molecular target drug can be calculated by using the number of photons N according to Equation (2).

The position Ro of the drug accumulation region 10 can be calculated from an intensity distribution of the numbers of photons of gamma rays detected by the gamma ray detecting elements 31a arranged along the circumference of the gantry opening 9.

On the other hand, the number of photons (which can be regarded as intensity) of a gamma ray detected by the gamma ray sensors 22i in the capsule medical apparatus 3 in a state in which the capsule medical apparatus 3 is inserted into a body cavity of the patient 2 as shown in Fig. 5 is as indicated by Expression (4).

$$Ic = N \times Sc/(\pi * L^2) \qquad (4)$$

Sc: a sectional area of a sensor surface of the gamma ray sensor (an area perpendicular to a straight line connecting the drug accumulation region and the capsule medical apparatus)
L: a distance from the drug accumulation region to the capsule medical apparatus

Sensor surfaces of the gamma ray sensors 22i provided in the capsule medical apparatus 3 are arranged to have isotropy as much as possible. This makes it possible to keep the sectional area Sc substantially constant irrespective of orientation of the capsule medical apparatus 3 during gamma ray detection (approximate the sectional area Sc as fixed irrespective of orientation).

A (linear) distance L from the drug accumulation region 10 to the capsule medical apparatus 3 can be calculated by substituting information concerning the number of photons N detected by the PET-CT apparatus 4 using Equation (3) into Equation (4).

Two unknown variables of the accumulation density $\rho$ and the distance L from the drug accumulation region 10 to the capsule medical apparatus 3 can be calculated by combining two medical apparatuses.

Equations (2) to (4) are equations formed on the premise that gamma ray detection by the PET-CT apparatus 4 and gamma ray detection by the capsule medical apparatus 3 are simultaneously performed. However, actually, in some cases, the gamma ray detections are performed in a different way.

For example, an examination by the capsule medical apparatus 3 may last about ten hours. Therefore, it is practically difficult to perform the gamma ray detection by the PET-CT apparatus 4 substantially simultaneously with the examination by the capsule medical apparatus 3.

Therefore, by using equations explained below, even if the gamma ray detections in the two medical apparatuses are not simultaneously performed, the accumulation density ρ and the distance L can be similarly calculated.

Since the number of positive electrons radiated from the radioactive isotope is attenuated as time elapses, it is necessary to calculate the number of positive electrons taking into account elapsed time from time of drug generation.

When the collapse speed P in Equation (2) is changed to collapse speed P(t) taking into account elapsed time t from the time of drug generation, the collapse speed P(t) is represented as indicated by Equation (5).

$$P(t) = Po \times exp(-\alpha * t) \quad (5)$$

Po: initial collapse speed (drug generation time)
$\alpha$: a collapse constant peculiar to nuclear species

When Equation (5) is taken into account, Equations (2), (3), and (4) are respectively changed to the following Equations (2'), (3'), and (4').

$$N = \rho \times P(t) \times 2 * T \quad (2')$$

$$I_p = \int N(t_1) \times h_p(R)/(\pi * (R-Ro)^2) dR$$
$$= \int \rho \times P(t_1) \times 2T \times h_p(R)/(\pi * (R-Ro)^2) dR \quad (3')$$

$$Ic = N(t_2) \times Sc/(\pi * L^2)$$
$$= \rho \times P(t_2) \times 2T \times Sc/(\pi * L^2) \quad (4')$$

$t_1$: elapsed time during PET image photographing
$t_2$: elapsed time during detection in the gamma ray sensor of the capsule medical apparatus

By using Equations (2') to (4'), it is possible to accurately calculate information concerning accumulation density and the distance L (from the drug accumulation region 10 to the capsule medical apparatus 3) taking into account information concerning the elapsed times $t_1$ and $t_2$ from the time of drug generation even if a time lag occurs in acquisition time of information concerning an examination (diagnosis) or a therapeutic procedure.

In the present embodiment, the estimation value Ies of intensity of a gamma ray detected at the predetermined distance Ls where the capsule medical apparatus 3 approaches the drug accumulation region 10 to a distance for enabling a therapeutic procedure by therapeutic light is calculated on the basis of Equations (3') and (4').

The intensity Ide detected by the capsule medical apparatus 3 and the estimation value Ies are compared to control an operation of the capsule medical apparatus 3.

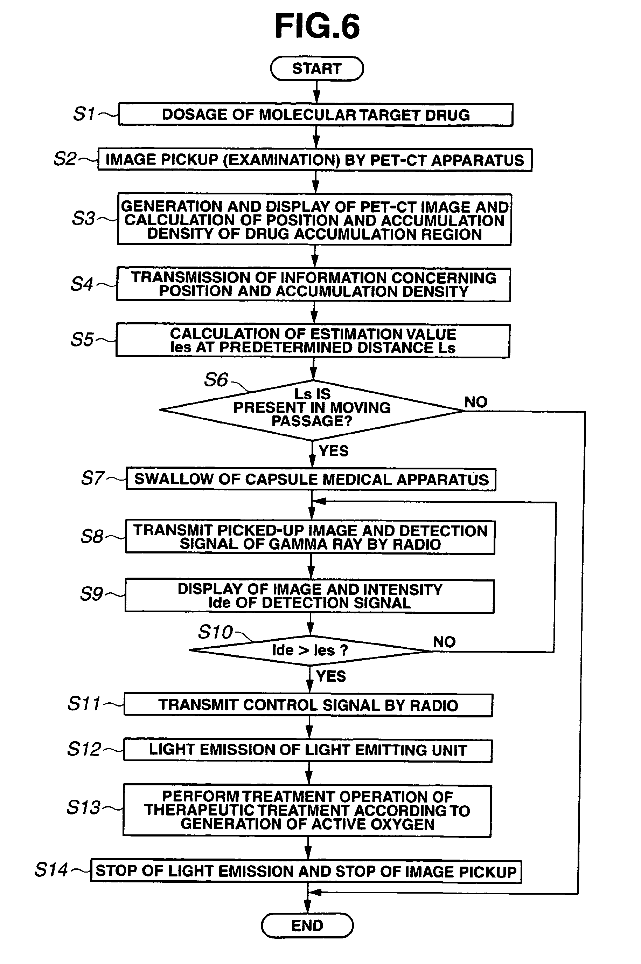

An operation related to a medical control method for performing treatment for a therapeutic procedure for a cancer tissue of the patient 2 according to the present embodiment is explained below with reference to Fig. 6.

In first step S1, a surgeon doses a molecular target drug as a specific drug to the patient 2 and accumulates the molecular target drug in the cancer tissue. The dosed molecular target drug accumulates in the drug accumulation region 10 as an accumulation region where the cancer tissue accumulates.

Subsequently, as shown in step S2, the surgeon performs image pickup of a PET image and image pickup (or an examination) of a CT image for the patient 2 using the PET-CT apparatus 4.

Specifically, the surgeon actuates the gantry for PET 11a and the gantry for CT 11b and performs image pickup of a PET image and image pickup of a CT image for the patient 2 placed on the bed 8 as shown in Fig. 1. In this case, the gamma ray detecting unit 31 in the PET-CT apparatus 4 detects a gamma ray emitted from the drug accumulation region 10 and outputs the gamma ray to the processing unit 12 as explained above.

As shown in step S3, the processing unit 12 generates a PET-CT image and displays the PET-CT image on the display unit 14. At the same time, the processing unit 12 calculates, for the entire body of the patient 2, information concerning both a position and accumulation density of the drug accumulation region 10 as the first living organism information. As shown in step S4, the processing unit 12 transmits, for example, the calculated information concerning the position and the accumulation density to the information processing apparatus 6 via the communication cable 5.

The information processing apparatus 6 records the received information in the information recording unit 16. In next step S5, the determination and control unit 47 of the information processing apparatus 6 calculates, using the received information, the estimation value Ies at the predetermined distance Ls corresponding to a case in which treatment for a therapeutic procedure is performed by the capsule medical apparatus 3 using therapeutic light.

In step S6, the determination and control unit 47 performs determination whether the predetermined distance Ls corresponding to the estimation value Ies is present in a moving passage in the patient 2 in the capsule medical apparatus 3.

Specifically, the determination and control unit 47 performs determination whether an area where intensity exceeding the estimation value Ies is detected is present in the moving passage in which the capsule medical apparatus 3 moves, in other words, an area equal to or smaller than the predetermined distance Ls is present in the moving passage. The determination may also be performed by the determining unit 47a.

When it is determined that the area is not present, the determination and control unit 47 displays indication to that effect on the display unit 17 and the processing operation shown in Fig. 6 ends.

On the other hand, when a determination result indicates that the predetermined distance Ls is present in the moving passage of the capsule medical apparatus 3, the determination and control unit 47 displays indication that the predetermined distance Ls is present on the display unit 17. In next step S7, the surgeon asks the patient 2 to swallow the capsule medical apparatus 3. As shown in step S8, the capsule medical apparatus 3 transmits, by radio, an (endoscope) image obtained by picking up an image while moving inside the body of the patient 2 and a detection signal obtained by detecting, with the gamma ray sensor 22i, a gamma ray as a radiation radiated from the drug accumulation region 10.

In step S9, the determination and control unit 47 displays the received (endoscope) image on the display unit 17 via the image generating unit 48 and displays the intensity Ide of the detection signal as the second living organism information.

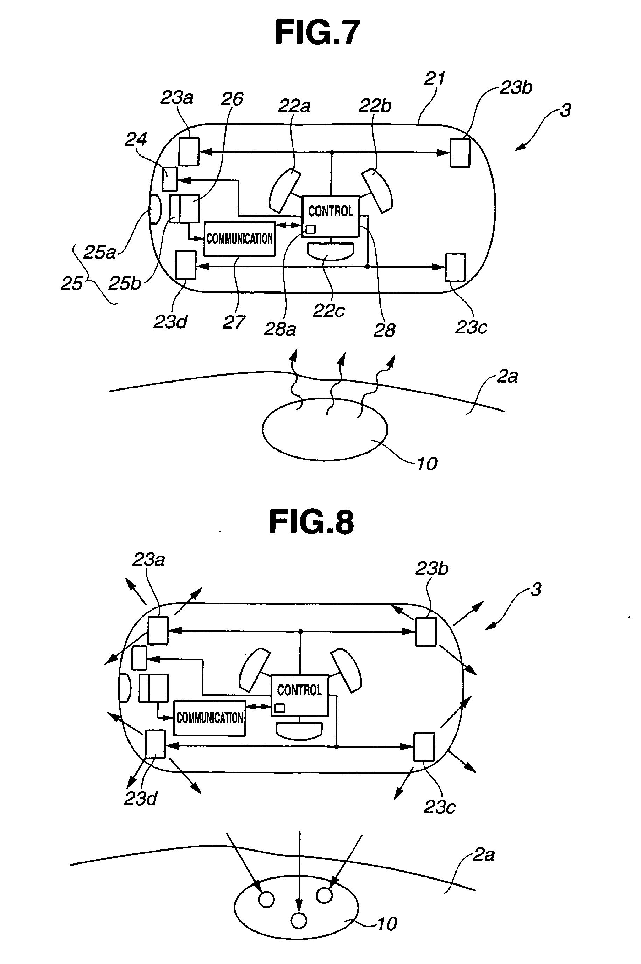

Fig. 7 shows a state in which the capsule medical apparatus 3 reaches near the drug accumulation region 10. The intensity Ide of the detection signal detected by the gamma ray sensor 22i increases when the capsule medical apparatus 3 passes near the drug accumulation region 10. In Fig. 7, reference sign 2a denotes a living organism tissue such as a digestive tract in the body cavity of the patient 2.

In step S 10, the determination and control unit 47 determines, with the determining unit 47a, whether the intensity Ide of the detection signal is equal to or larger than the estimation value Ies, i.e., satisfies Expression (1).

When a determination result indicates that the intensity Ide does not satisfy Expression (1), the operation returns to the processing in step S8. When a determination result indicates that the intensity Ide satisfies Expression (1), the operation proceeds to next step S 11.

In step S11, the determination and control unit 47 transmits a control signal to the capsule medical apparatus 3 via the communication unit 45. In step S12, when the capsule control unit 28 of the capsule medical apparatus 3 receives the control signal, the capsule control unit 28 performs control for causing the light emitting unit 23j to emit light.

As shown in step 513, according to the light emission of the light emitting unit 23j, the photosensitive substance contained in the molecular target drug in the drug accumulation region 10 generates active oxygen and performs a treatment operation of a therapeutic procedure. Fig. 8 shows a state in which, in response to the control signal, the light emitting units 23j change to a light emitting state. In Fig. 8, for simplification, reference numerals of the capsule control unit 28 and the like are omitted.

The drug accumulation region 10 is irradiated by therapeutic light from the light emission of the light emitting units 23j. The photosensitive substance in the drug accumulation region 10 generates active oxygen 52 (white circles in Fig. 8) according to the irradiation by the therapeutic light. The active oxygen 52 acts to kill cells of the cancer tissue in which the molecular target drug accumulates.

When the capsule control unit 28 causes the light emitting units 23j to emit light in response to the control signal, the capsule control unit 28 may monitor intensity of a detection signal of the gamma ray sensors 22i, selectively cause the light emitting unit suitable for irradiation of the drug accumulation region 10 to emit light, and control a light emission amount.

In step S 13, the capsule control unit 28 causes the light emitting units 23j to emit light for a predetermined time. Thereafter, as shown in step S 14, the capsule control unit 28 stops the light emission. Further, the capsule control unit 28 performs control for stopping the image pickup of the image pickup unit 25 and the detection operation of the gamma ray sensors 22i. The capsule control unit 28 ends the operation shown in Fig. 6.

In this way, according to the first embodiment, it is possible to perform a therapeutic procedure from an inside of a living organism using the capsule medical apparatus 3 on the basis of position and density information of the molecular target drug obtained by the PET-CT apparatus 4.

Therefore, when a therapeutic procedure is applied to a lesion region such as a cancer tissue formed in, for example, an organ present in a remote position from a body surface, it is unnecessary to radiate a radiation or the like from an outside of the living organism. A more efficient therapeutic procedure can be performed with low energy when the lesion region is treated by using an internal medical apparatus that can be arranged in a position closer to the lesion region as in the present embodiment.

In the present embodiment, it is possible to directly approach a found lesion region and accurately perform a therapeutic procedure.

Further, since the number of times the drug is dosed to the patient 2 can be reduced by simultaneously performing an examination and a therapeutic procedure of the entire body by dosing the drug once, the leads to a reduction in examination and therapeutic procedure time.

In the present embodiment, gold nano-particles as particulates of gold, carbon nano-particles as particulates of carbon, or the like may be added to the molecular target drug. This makes it possible to cause the gold nano-particles or the like to efficiently generate heat according to radiation of light and show a thermal therapeutic procedure effect for a lesion tissue near the molecular target drug to which the particulates are added.

As in the present embodiment, when a therapeutic procedure is performed by the light emitting units 23j using, for example, therapeutic light in a visible to near infrared region, since reaching depth of light to a living body tissue in a lesion region is relatively small, an effective therapeutic procedure effect can be shown for a lesion in a digestive tract mucosa surface layer. However, in some cases, a procedure for a deeper living organism tissue is desired.

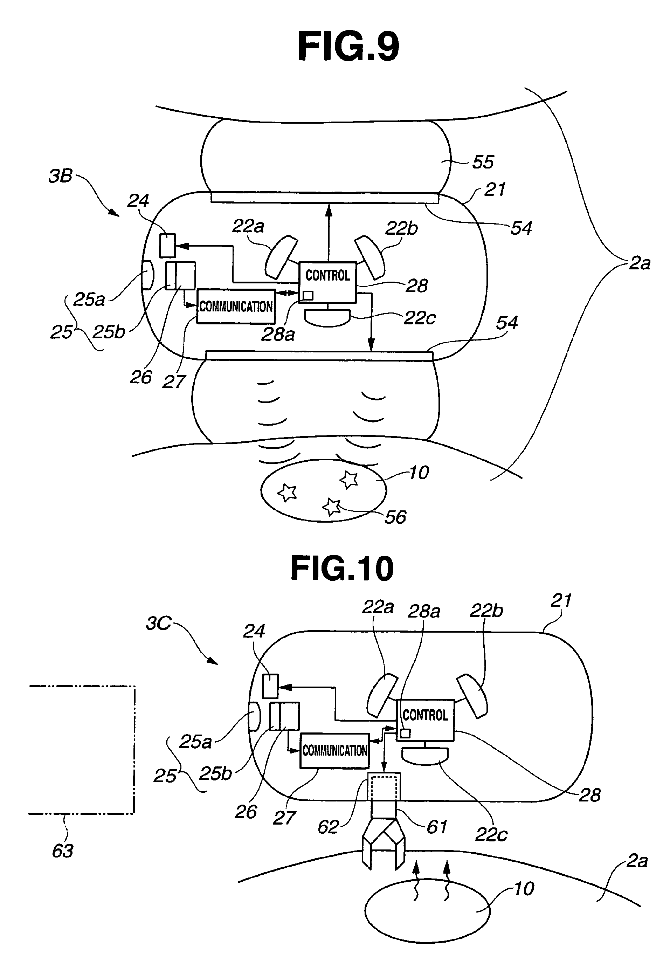

To make it possible to cope with such a case, a medical system of a modification obtained by modifying the capsule medical apparatus 3 in the medical system 1 according to the first embodiment may be configured. Like a capsule medical apparatus 3B configuring a modification shown in Fig. 9, ultrasound having larger reaching depth to the drug accumulation region 10 may be radiated to perform a therapeutic procedure.

The medical system in the modification has a configuration in which the capsule medical apparatus 3B shown in Fig. 9 including ultrasonic oscillators 54 is provided instead of the capsule medical apparatus 3 in Fig. 1.

In the capsule medical apparatus 3B shown in Fig. 9, as the operation unit, the ultrasonic oscillators 54 are arranged along a circumferential direction of the armor container 21 instead of the light emitting units 23j in the capsule medical apparatus 3 in the medical system 1 according to the first embodiment explained above.

A balloon 55 is arranged along the circumferential direction of the armor container 21. The capsule control unit 28 also performs control for expanding (inflating) and shrinking the balloon 55.

The ultrasonic oscillators 54 are dividedly arranged along the circumferential direction of the armor container 21. A direction in which ultrasound is emitted can be changed and set by the capsule control unit 28. The other components are the same as those of the capsule medical apparatus 3.

A procedure of a medical control method of the modification is explained below. Since the procedure is similar to Fig. 6 in the first embodiment, only differences are explained with reference to Fig. 6.

In the modification, as the molecular target drug dosed to the patient 2 in step S1 in Fig. 6, a drug including, rather than the photosensitive substance, for example, polymer micelles 56 containing a therapeutic agent for a lesion region is dosed.

The processing in step S2 and subsequent steps in Fig. 6 are sequentially performed up to step S 11. When the capsule control unit 28 receives the control signal by radio in step S11 in Fig. 6, the capsule control unit 28 inflates the balloon 55 as shown in Fig. 9.

Instead of causing the light emitting units 23j to emit light in step S12 in Fig. 6, the capsule control unit 28 controls the ultrasonic oscillators 54 to radiate ultrasound, destroys the polymer micelles 56 with the ultrasound, and disperses the therapeutic agent contained in the polymer micelles 56. It is possible to perform an operation of a therapeutic procedure for, for example, a cancer tissue as a lesion region using the dispersed therapeutic agent.

When a therapeutic procedure target is, for example, a thrombus rather than the lesion region such as the cancer cells, a blood dissolution agent or the like may be contained by the polymer micelles 56. Then, similarly, it is possible to destroy the polymer micelles 56 with irradiation by ultrasound, dissolve blood in a thrombus portion with the blood dissolution agent seeping from the polymer micelles 56, and perform a therapeutic procedure for the thrombus.

(Second Embodiment)

Fig. 10 is shows a configuration of a capsule medical apparatus 3C in a second embodiment of the present invention in an operation explanatory diagram of the capsule medical apparatus 3C. In the present embodiment, the capsule medical apparatus 3C shown in Fig. 10 is adopted instead of the capsule medical apparatus 3 in the medical system 1 shown in Fig. 1.

The capsule medical apparatus 3C includes a clip 61 that freely projects from the armor container 21 and disengages from the capsule medical apparatus 3C.

When the capsule control unit 28 receives a control signal from the information processing apparatus 6, the capsule control unit 28 controls a clip driving unit 62 to drive to project the clip 61 from the capsule medical apparatus 3C. The clip 61 is formed of, for example, a shape memory substance. When the clip driving unit 62 is driven to generate heat by the capsule control unit 28, the clip 61 in a contracted state expands and projects to the outside from a clip housing section as shown in Fig. 10.

The projecting clip 61 sticks in the drug accumulation region 10 as a specific region in the living organism tissue 2a in the body cavity of the patient 2 and fixed. In other words, the fixed clip 61 is a label indicating the drug accumulation region 10. In this way, in the present embodiment, marking means for attaching a label to the drug accumulation region 10 is formed by the clip 61 and the clip driving unit 62 as an operation unit.

In the present embodiment, in addition to the function of controlling the operation of the gamma ray sensors 22i as the detecting unit for acquiring living organism information, the capsule control unit 28 controls an operation for attaching (retaining) the clip 61 to the drug accumulation region 10. In this way, in the configuration of the present embodiment, the capsule medical apparatus 3 in the first embodiment is only changed to the capsule medical apparatus 3C.

In other words, the capsule medical apparatus 3C in the present embodiment is a capsule medical apparatus that performs an operation for attaching a label to a specific region of the living organism tissue 2a using the first living organism information by the PET-CT apparatus 4. It is assumed that the capsule medical apparatus 3C doses the molecular target drug including the positive electron to the patient 2.

Actions of the gamma ray sensors 22i and the communication unit 27 included in the capsule medical apparatus 3C are equivalent to the actions in the first embodiment. Reception of information from the PET-CT apparatus 4 in the information processing apparatus 6, determination of a distance between the capsule medical apparatus 3C and the drug accumulation region 10, and the like are equivalent to the reception, the determination, and the like in the first embodiment.

When it is determined that the capsule medical apparatus 3C in the second embodiment is close to the drug accumulation region 10, the capsule control unit 28 receives a control signal corresponding to a determination result from the information processing apparatus 6 and controls the clip 61 to be retained as a labeling object near the drug accumulation region 10 in the living organism.

The labeling object may be the clip 61 shown in Fig. 10 that clips and fixes a portion of the living organism tissue 2a. However, the labeling object is not limited to this and may be a stent or the like retained in a digestive tract. A method of diffusing a drug as a label may be adopted.

When the drug is diffused, a drug that is retained for a relatively long period near the drug accumulation region 10 of a cancer tissue or the like as a target of a therapeutic procedure or the like is desirable.

The clip 61 or a marker such as a drug serving as the label is desirably an object that can also be imaged in the PET-CT apparatus 4.

In the present embodiment, an endoscope 63 having a tubular inserting portion is inserted to the drug accumulation region 10 attached with a marker or the like to apply treatment for a therapeutic procedure to the drug accumulation region 10 of a cancer tissue or the like attached with the marker using a treatment instrument or performs treatment for sampling a tissue (biopsy) and performs pathologic diagnosis of a lesion tissue.

With the capsule medical apparatus 3C according to the second embodiment, since the labeling object is retained or the marker is attached near the drug accumulation region 10, there is an advantage that it is easy to endoscopically approach a lesion region such as a cancer tissue detected by the PET-CT apparatus 4.

An operation in this case is substantially the same as the operation in the first embodiment from steps S1 to S11 in the flowchart shown in Fig. 6.

However, in the present embodiment, the molecular target drug in step S1 only has to be a drug not containing a photosensitive substance. In step S 11 after passing through the steps S2 to S10, the determination and control unit 47 transmits a control signal to the capsule medical apparatus 3C by radio.

In next step, when the capsule control unit 28 receives the control signal, the capsule control unit 28 actuates the clip driving unit 62 and fixes (retains) the clip 61 near the drug accumulation region 10.

For example, the information processing apparatus 6 records a position of a label imaged by the PET-CT apparatus 4 and the drug accumulation region 10 as images and renders the drug accumulation region 10 as a three-dimensional image of a biopsy target region to generate a pseudo endoscope image.

This makes it possible to accurately reproduce a positional relation between a retained place where the label is retained and a place detected as the drug accumulation region 10.

Therefore, during an endoscope examination performed later, it is possible to insert a distal end portion of the endoscope 63 to the place where the labeling object is retained and perform, with reference to the three-dimensional image, a biopsy with the place detected as the drug accumulation region 10 set as a biopsy target.

Therefore, it is unnecessary to dose a drug again to the drug accumulation region 10 detected by the PET-CT apparatus 4 using the molecular target drug, in other words, a lesion region such as a cancer tissue. There is an effect that a lesion tissue can be accurately sampled and pathologic diagnosis can be performed.

According to the present embodiment, a label can be attached to a target region simultaneously with an examination by the PET-CT apparatus 4 by the capsule medical apparatus 3C. Therefore, there is an effect that the target region can be easily found in an endoscope examination performed later and perform a biopsy or the like in a short time and smoothly.

(Third Embodiment)

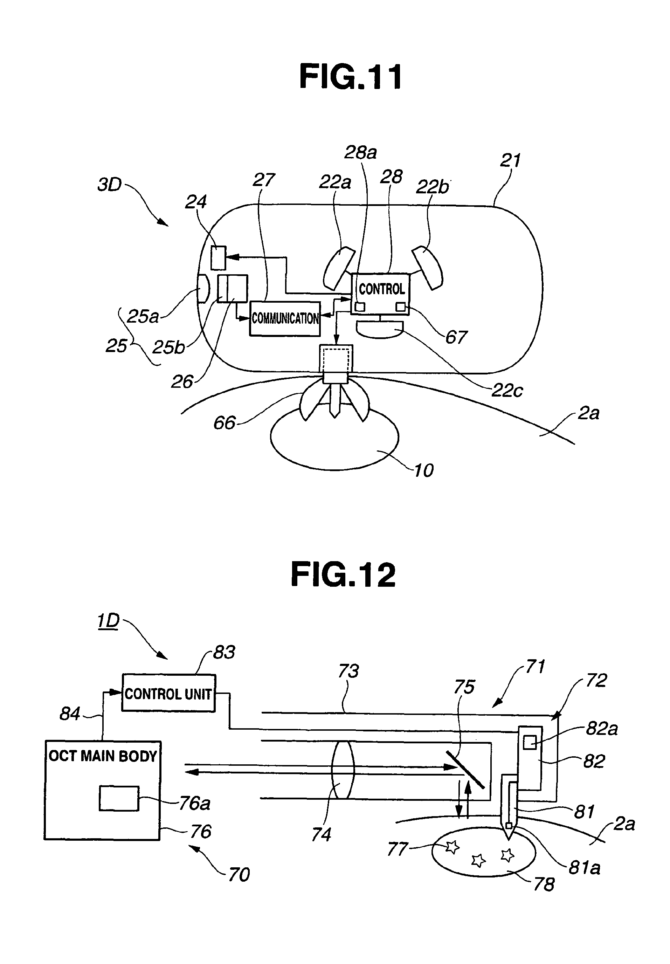

Fig. 11 shows a configuration of a capsule medical apparatus 3D in a third embodiment of the present invention in an operation explanatory diagram.

The capsule medical apparatus 3D according to the present embodiment includes biopsy forceps (a biopsy treatment instrument) 66, which directly samples a living organism tissue from the living organism tissue 2a, as an operation unit as shown in Fig. 11 instead of the marking means for attaching a label to a specific region of the living organism tissue 2a in the capsule medical apparatus 3C according to the second embodiment.

The capsule control unit 28 in the present embodiment has a control function same as the control function in the first embodiment and has a function of a biopsy forceps control unit 67 for performing control of an operation for sampling a tissue (biopsy) as a predetermined operation by the biopsy forceps 66.

An operation in the present embodiment is the same as the operation in steps S1 to S11 in Fig. 6 related to the operation or the medical control method in the first embodiment. Differences are explained below.

A characteristic of the capsule medical apparatus 3D in the present embodiment is that, when the information processing apparatus 6 determines that the capsule medical apparatus 3D is close to the drug accumulation region 10, the capsule control unit 28 performs control to directly sample a tissue from the living organism tissue 2a using the biopsy forceps 66 in response to a determination result from the information processing apparatus 6. Therefore, according to the present embodiment, there is an effect that a tissue can be sampled from a lesion region such as a cancer tissue.

The biopsy forceps 66 need to surely perform tissue sampling from the drug accumulation region 10 found by the PET-CT apparatus 4. Therefore, it is desirable to use a material that can be imaged by an X-ray as a material of the biopsy forceps 66 such that a positional relation between the biopsy forceps 66 and the drug accumulation region 10 can be detected by the PET-CT apparatus 4.

It is more desirable to provide, at a distal end portion of the biopsy forceps 66, a sensor for detecting presence or absence of a drug. When a distal end of the biopsy forceps 66 comes into contact with a region near the drug accumulation region 10, if control for performing tissue sampling is performed only when a signal level detected by the sensor is equal to or higher than a predetermined level (of the drug accumulation region 10), it is possible to reduce a biopsy from an unnecessary tissue and surely sample a tissue of a biopsy target.

According to the third embodiment, it is possible to accurately guide the capsule medical apparatus 3D to a lesion region that should be subjected to a biopsy and apply a sure biopsy to a living organism tissue of the lesion region.

Since the sensor is provided in the biopsy forceps 66 themselves, it is possible to perform sure tissue sampling from the living organism tissue of the lesion region.

In the first to third embodiments, the combinations with the PET-CT apparatus 4 are mainly explained. However, medical apparatuses to be combined are not limited to the case. For example, in the example explained above, the PET-CT apparatus 4 is used as the external medical apparatus in the first embodiment. However, a PET apparatus not including a CT apparatus section may be used.

A combination with, for example, a SPECT (Single Photon Emission Computed Tomography) as a medical apparatus that can be combined with the molecular target drug or an MRI apparatus that makes use of a magnetic resonance phenomenon cay be used instead of the PET-CT apparatus 4 as the external medical apparatus.

When the capsule medical apparatus 3D is used in combination with the MRI apparatus, a molecular target drug that can be detected by the magnetic resonance phenomenon is dosed to a living organism.

An MR signal (a radio wave) generated by magnetic resonance is detected by an MR signal (radio wave) sensor as the second detecting unit provided instead of the gamma ray sensors 22i provided in the capsule medical apparatus 3. Intensity of the MR signal as the second living organism information is acquired from a signal level of the detected information.

A therapeutic procedure operation is controlled on the basis of the information concerning the intensity obtained by the MR signal sensor and the information concerning the position and the accumulation density of the drug accumulation region (as the first living organism information) generated from the detected information by the first detecting unit by the MRI apparatus arranged outside the body. Consequently, there is an effect that it is possible to directly approach a lesion region such as a cancer tissue and accurately perform a therapeutic procedure and reduce examination and therapeutic procedure time.

In the explanation of the first to third embodiments, the same signal of, for example, the gamma ray as a radiation is detected by the PET-CT apparatus 4 and the capsule medical apparatus. However, the capsule medical apparatus may detect a physical signal different from a signal detected by the PET-CT apparatus 4.

For example, it is also possible that fluorescent pigment generally used in combination with an endoscope is combined with the molecular target drug and fluorescent light generated in response to excitation light radiated from the capsule medical apparatus is captured by an image pickup device of the capsule medical apparatus.

Besides, there are various detecting methods such as detection that makes use of an increase in reflectance in a specific wavelength band due to surface enhanced Raman scattering or surface plasmon. A capsule medical apparatus that utilizes signals of these methods may be adopted.

(Fourth Embodiment)

A fourth embodiment of the present invention is explained with reference to Fig. 12. Fig. 12 shows a schematic configuration of a medical system 1D according to the fourth embodiment of the present invention in an operation explanatory diagram of the medical system 1D.

The medical system 1D according to the present embodiment includes an OCT (optical coherent tomography) apparatus 70 including an OCT probe 71 as a first medical apparatus inserted into (arranged in) a patient (a living organism) and a radio current probe 72 as a second medical apparatus attached to a distal end side of the OCT probe 71.

In other words, the medical system 1D according to the present embodiment is a system including two medical apparatuses having different functions of acquiring (detecting) different first and second kinds of living organism information and both inserted into the living organism.

The OCT probe 71 has an elongated and tubular insertion section 73 inserted into the patient. A lens 74 that condenses light and a scanning mirror 75 that scans the light are arranged on a distal end side of the insertion section 73 as a first detecting unit for acquiring a position of an accumulation region 78 wherein a cancer tissue as first living organism information accumulates.

An OCT main body 76 provided outside the living organism on a proximal end side of the insertion section 73 includes a not-shown light source in the OCT main body 76. Low-coherency light having small coherent length (i.e., small interfering distance) emitted from the light source is transmitted to the distal end side of the insertion section 73.

The transmitted low-coherency light is emitted in a direction perpendicular to an axis of an insertion section 73 via the lens 74 and the scanning mirror 75. The scanning mirror 75 is driven to rotate around the axis of the insertion section 73 or driven to swing in an appropriate angle range.

In this case, a substance such as gold nano-particles 77 packaged by protein that specifically combines in cancer cells is dosed in advance to the patient. As shown in Fig. 12, the gold nano-particles 77 accumulate in a region (the accumulation region 78) where the cancer tissue accumulates in the living organism tissue 2a (inside the patient).

The low-coherency light radiated on the accumulation region 78 as such is intensely reflected by the gold nano-particles 77 accumulated in the accumulation region 78.

The reflected light returns to the OCT main body 76 on the proximal end side of the insertion section 73 via the scanning mirror 75. A not-shown reference light generating unit that generates reference light is provided in the OCT main body 76. Interference occurs within a range in which optical path length of the light returning from the scanning mirror 75 side and optical path length of the reference light generating unit substantially coincides with each other.

The optical path length of the reference light is changed at, for example, a predetermined period. According to the periodical change of the optical path length of the reference light, among scattered lights of the low-coherency light radiated on the living organism tissue 2a, light at a distance coinciding with an amount of the periodical change of the optical path length from the scanning mirror 75 is detected as interference light.

As explained above, since the gold nano-particles 77 include metal particles, the gold nano-particles 77 generate intense backscattering light. The intense backscattering light is detected as intense interference light.

The interference light is photoelectrically converted into an interference signal by a photodetector in the OCT main body 76 and inputted to a signal processing circuit 76a in the OCT main body 76. The signal processing circuit 76a generates an OCT tomography image with intensity of the interference signal set as luminance. The signal processing circuit 76a has a function of generating first living organism information from detected information by the first detecting unit and performs processing for calculating a position of the accumulation region 78 from the interference signal.

On the other hand, a current needle 81 having a diameter of, for example, about $\phi$1 mm that generates a radio wave and a radio current probe 72 including a driving unit 82 that drives the current needle 81 to freely project from an opening at a distal end portion are arranged in a position close to the scanning mirror 75 at a distal end portion of the insertion section 73.

The driving unit 82 incorporates a radio wave generating unit 82a as an operation unit that performs an operation for causing the current needle 81 to generate a radio wave. The driving unit 82 is connected to a control unit 83 provided on the proximal end side of the insertion section 73 by a signal line. The control unit 83 performs control for applying the radio wave to the current needle 81 via the driving unit 82 and causing the current needle 81 to generate a radio wave.

A temperature sensor 81a that detects temperature is provided at a distal end of the current needle 81 as a second detecting unit for acquiring the second living organism information. Detected information (a signal level) detected by the temperature sensor 81a is temperature information of the accumulation region 78 as the second living organism information. The temperature information is sent to the control unit 83 via the driving unit 82.

The control unit 83 may control the temperature sensor 81a to output detected temperature information only when the current needle 81 is projected by the driving unit 82 and the distal end of the current needle 81 comes into contact with or punctured into the living organism tissue 2a near the accumulation region 78.

The control unit 83 is connected to the OCT main body 76 via a communication cable 84. Position information of the accumulation region 78 in an OCT tomography image is inputted from the OCT main body 76 side as the first living organism information.

When the accumulation region 78 is captured within a scanning range (or the OCT tomography image) by the OCT probe 71 displayed as the OCT tomography image, the control unit 83 determines that the distal end of the OCT probe 71 is present at a distance close to the accumulation region 78 (in an area suitable for treatment).

When information concerning the accumulation region 78 captured within the scanning range (or the OCT tomography image) and information concerning the position of the accumulation region 78 are inputted from the OCT main body 76, the control unit 83 performs control for projecting the current needle 81 to the driving unit 82, control for causing the current needle 81 to output a radio wave, and control of a direction in which the radio wave is outputted, i.e., directivity.

Fig. 12 shows a state in which the driving unit 82 that receives a control signal from the control unit 83 projects the current needle 81 from the distal end portion, punctures the current needle 81 into the living organism tissue 2a, and radiates a radio wave.

The gold nano-particles 77 that receive the radio wave generates heat and the accumulation region 78 is heated. In this case, temperature of the accumulation region 78 is detected by the temperature sensor 81a and sent to the control unit 83. The control unit 83 controls an output level of the radio wave according to the information from the OCT main body 76 and temperature information.

The control unit 83 controls the output level according to the temperature information detected by the temperature sensor 81a to keep temperature suitable for killing cancer cells in the accumulation region 78 and properly performs a cancer therapeutic procedure.

When there is the penetrating current needle 81 within the scanning range displayed as the OCT tomography image, a penetrating position of the current needle 81 can be specified according to interference light by a scattering light component from a surface of the current needle 81 in the OCT tomography image.

For example, the signal processing circuit 76a of the OCT main body 76 calculates a positional relation between the accumulation region 78 of the gold nano-particles 77 and the current needle 81 on the basis of the OCT tomography image by an interference signal acquired in the OCT main body 76 at the point. The signal processing circuit 76a sends information concerning the positional relation to the control unit 83. The control unit 83 corrects a projection amount of the current needle 81 and makes it easy to more appropriately perform treatment for the cancer cells in the accumulation region 78.

The control unit 83 stops the radiation of the radio wave after time in which it is estimated that the cancer therapeutic procedure can be properly performed and performs control for housing the current needle 81 in the distal end portion.

In a configuration example shown in Fig. 12, the control unit 83 may be arranged in the living organism. Information of the OCT 70 may be transmitted to the control unit 83 by radio.

According to the present embodiment in which such actions are performed, as in the case of the medical apparatus arranged outside the living organism and the medical apparatus in the living organism in the first embodiment, it is possible to detect a lesion region such as a cancer tissue using the two medical apparatuses inserted into the living organism and smoothly and properly apply treatment for a therapeutic procedure to the detected lesion region.

Specifically, according to the present embodiment, when the distal end portion of the OCT probe 71 is captured in the OCT tomography image, the control unit 83 determines that the distal end portion of the OCT probe 71 is present in an area suitable for treatment close to the lesion region, projects the current needle 81 of the radio current probe 72 provided near the distal end portion, penetrates the current needle 81 into the lesion region, and performs control for causing the current needle 81 to output a radio wave. This makes it possible to cause the gold nano-particles 77 accumulated in the lesion region to generate heat and perform treatment for a therapeutic procedure for killing cancer cells of the lesion region.

According to the present embodiment, a positional relation between the lesion region (the accumulation region 78) where the gold nano-particles 77, which are caused to generate heat to perform a therapeutic procedure, accumulate and the current needle 81 is calculated from the OCT tomography image and a projection amount of the current needle 81 that outputs a radio wave is corrected according to information concerning the positional relation. This makes it possible to properly perform treatment for a therapeutic procedure for the lesion region.

According to the present embodiment, when the gold nano-particles 77 are caused to generate heat to perform treatment for a therapeutic procedure, temperature of the lesion region is detected by the temperature sensor 81 a provided at the distal end of the current needle 81, an output level of a radio wave is controlled according to information concerning the temperature, and temperature suitable for treatment of the lesion region is kept. This makes it possible to perform an efficient therapeutic procedure.

It is also possible to perform control of directivity to orient a direction for outputting the radio wave to the lesion region according to information concerning a positional relation between the lesion region and the current needle 81. It is possible to efficiently apply treatment for a therapeutic procedure by the radio wave to the lesion region.

In the present embodiment, the case of the OCT apparatus 70 and the radio current probe 72 is explained. However, the present invention is not limited to this case and may be, for example, a combination of a normal endoscope and a capsule medical apparatus (or a capsule endoscope). Or it may be a combination of a normal endoscope and a retainer (sensor) in the body or a combination of an ultrasonic probe, a confocal probe, or the like, a needle sensor, and an endoscope. The two medical apparatuses both inserted into the body may be any medical apparatuses as long as the medical apparatuses can be inserted into the body.

(Fifth Embodiment)

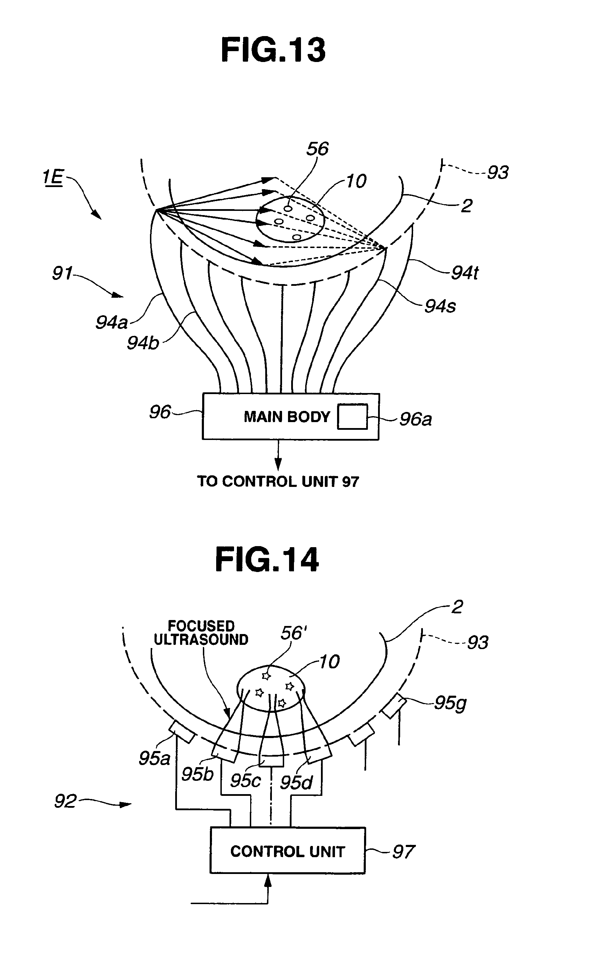

A fifth embodiment of the present invention is explained below with reference to Figs. 13 and 14. Fig. 13 shows a schematic configuration of an optical mammography apparatus 91 included in a medical system 1E according to the fifth embodiment of the present invention. Fig. 14 shows a schematic configuration of an ultrasonic apparatus 92. The medical system 1E according to the present embodiment is a medical system including two medical apparatuses both arranged outside a living organism and including a first detecting unit and a second detecting unit for respectively acquiring first living organism information and second living organism information.

As shown in Fig. 13, the optical mammography apparatus 91 includes a gantry 93 in which a subject region (e.g., a breast) of the patient 2 is housed. One ends of plural optical fibers 94a, 94b, ..., 94s, and 94t are arranged along a circumference of the gantry 93.

The other ends of the optical fibers 94k (k = a, b, ..., and t) are connected to an optical mammography main body (simply referred to as main body) 96. A light emitting unit and a light receiving unit are provided in the main body 96. As shown in Fig. 13, the main body 96 radiates light from, for example, one end of the optical fiber 94a to an inner side of the gantry 93. The main body 96 has a function of a first detecting unit for receiving, with another optical fiber, in Fig. 13, for example, the optical fiber 94s, scattered lights of the radiated light and acquiring (detecting) the first living organism information by scattering of light.

A molecular target drug targeting cancer cells is dosed in advance to the patient 2. In the molecular target drug, a cancer therapeutic agent is contained in the polymer micelles 56. The polymer micelles 56 have a predetermined absorption coefficient or a scattering coefficient with respect to light having specific wavelength. As explained above, the molecular target drug accumulates in a cancer tissue as the drug accumulation region 10.

In the gantry 93, the ultrasonic apparatus 92 is provided in which a large number of (plural) ultrasonic transducers 95a, 95b, 95c, 95d, ..., and 95g are arranged as shown in Fig. 14 adjacent to arrangement positions of the optical fibers 94a, 94b, ..., and 94t.

In Fig. 13, a state in which light emission and light reception are performed by one optical fiber 94a and one optical fiber 94s is shown. However, light emission and light reception are performed by using plural optical fibers. An arithmetic processing unit 96a in the main body 96 performs an arithmetic operation for calculating a distribution of the scattering coefficient and the absorption coefficient in a living organism tissue of the patient 2 on an inner side of the gantry 93 from information concerning arrangement positions of the plural optical fibers and scattering lights.

According to the arithmetic operation by the arithmetic processing unit 96a, a three-dimensional image is configured and information concerning a position of the drug accumulation region 10 of the molecular target drug is calculated (generated) as the first living organism information from the established three-dimensional image.

The position information of the drug accumulation region 10 generated by the arithmetic processing unit 96a in the main body 96 is transmitted to an ultrasonic control unit (abbreviated as control unit) 97 of the ultrasonic apparatus 92 shown in Fig. 14.

An operation of the ultrasonic transducers 95l (l = a, ..., and g) as operation units that perform radiation of ultrasound is controlled by the control unit 97. The control unit 97 performs control for applying, from a not-shown transmitting unit in the control unit 97, an ultrasonic driving signal for causing the ultrasonic transducers 95l to generate ultrasound.

The ultrasonic transducer 95l applied with the ultrasonic driving signal radiates ultrasound that focuses toward an inside of the gantry 93.

In this case, the control unit 97 selects, on the basis of the position information of the drug accumulation region 10 received from the main body 96, the ultrasonic transducers 95l (in an example shown in Fig. 14, three ultrasonic transducers 95b to 95d), which are actually driven, such that the ultrasound is focused and radiated on the drug accumulation region 10.

The ultrasonic apparatus 92 destroys the polymer micelles 56 in the drug accumulation region 10 with the radiation of the focused ultrasound and performs an operation for treatment of a cancer therapeutic procedure (in Fig. 14, the broken polymer micelles are indicated by 56').

The ultrasonic transducers 951 included in the ultrasonic apparatus 92 also plays a role of microphones as second detecting units for acquiring information concerning ultrasonic energy radiated on the drug accumulation region 10 as the second living organism information. The ultrasonic transducers 951 detects echo sound generated when ultrasound is radiated on the patient 2. Information concerning the detected echo sound is inputted to the control unit 97.

The control unit 97 generates, from the detection information of the echo sound, an estimation value of the information concerning the ultrasonic energy radiated on the drug accumulation region 10 (an amplitude value and a frequency of ultrasonic vibration).

The control unit 97 controls, using the position information of the drug accumulation region 10 obtained by the optical mammography apparatus 91 and the information concerning the ultrasonic energy obtained by the ultrasonic apparatus 92 as control information, an ultrasonic output applied to the ultrasonic transducers 951 and a radiation direction of ultrasound on the basis of the control information.

The control unit 97 can efficiently cure, by performing the control in this way, the cancer tissue in the drug accumulation region 10 as an affected region or a lesion region, which should be cured, without affecting other tissues.

The control unit 97 can control transmission and reception of ultrasound by the plural ultrasonic transducers 951 and generate (acquire), with the plural ultrasonic transducers 951, position or distance information by the ultrasound with respect to the drug accumulation region 10 as the second living organism information. In this case, the ultrasonic transducers 95I have a function of the second detecting units for acoustically acquiring a position or a distance of the drug accumulation region 10.

The control unit 97 may control, using information by the function of the second detecting units of the ultrasonic transducer 951, magnitude of ultrasonic energy in performing a therapeutic procedure. For example, when the drug accumulation region 10 is detected in a position close to a body surface, the control unit 97 may perform control to reduce an ultrasonic energy amount radiated to perform a therapeutic procedure.

By using the acoustic information besides optical information by the optical mammography apparatus 91 in this way, there is an effect that it is possible to more accurately detect the drug accumulation region 10 and appropriately perform treatment for a therapeutic procedure using ultrasound.

If the molecular target drug has a structure in which an optical characteristic changes according to the destruction of the polymer micelles 56, it is possible to also perform monitoring of a therapeutic procedure effect using the optical mammography apparatus 91.

For example, when a light absorption coefficient of the molecular target drug is increased by the destruction of the polymer micelles 56, the monitoring of a therapeutic procedure effect is performed as explained below.

When ultrasonic energy for performing a therapeutic procedure is radiated on the drug accumulation region 10 as a lesion region for a predetermined time, it is possible to estimate the number of the polymer micelles 56 destroyed in the drug accumulation region 10 from an amount of decrease in light intensity detected by the optical mammography apparatus 91 before and after the radiation.

Therefore, a surgeon can estimate, from an estimated value of the number of the destroyed polymer micelles 56, whether an amount of the cancer therapeutic agent flown out from the polymer micelles 56 because of the destruction is an amount suitable for a therapeutic procedure. The surgeon can control ultrasonic energy to be radiated such that the amount of the cancer therapeutic agent is suitable for the therapeutic procedure.

Further, a temporal change in the light intensity detected by the optical mammography apparatus 91 is monitored. It is estimated that, when a cancer tissue in the lesion region is killed by the cancer therapeutic agent and the therapeutic procedure progresses, accumulation density of the molecular target agent accumulating in the lesion region falls. Therefore, by monitoring the temporal change in the light intensity detected by the optical mammography apparatus 91, it is possible to use the temporal change in the light intensity for estimation of a therapeutic procedure effect.

According to the present embodiment in which such actions are performed, as in the case of the medical apparatus arranged outside the living organism and the medical apparatus in the living organism in the first embodiment, it is possible to detect a lesion region such as a cancer tissue using the two medical apparatuses both arranged outside the living organism and smoothly and properly apply treatment for a therapeutic procedure to the detected lesion region.

Specifically, according to the present embodiment, a position of the drug accumulation region 10 accumulated in the lesion region is detected by the optical mammography apparatus 91. The control unit 97 of the ultrasonic apparatus 92 controls, on the basis of information concerning the position, driving of the transducers 951 such that ultrasound is focused and radiated on the drug accumulation region 10. Therefore, it is possible destroy the polymer micelles 56 accumulated in the drug accumulation region 10 by the radiation of the ultrasound, and smoothly perform treatment for a therapeutic procedure for the lesion region with the therapeutic agent contained in the polymer micelles 56.

The control unit 97 estimates information concerning radiated ultrasonic energy from information concerning echo sound of the ultrasound radiated on the drug accumulation region 10 and controls, using the estimated information concerning the ultrasonic energy as control information, an ultrasonic output of the transducers 95I in radiating the ultrasound on the drug accumulation region 10 and a radiation direction of the ultrasound. This makes it possible to efficiently cure the lesion region that should be cured.

The control unit 97 can control magnitude of the ultrasonic energy in performing a therapeutic procedure using information concerning a position or a distance of the drug accumulation region 10 by the transducers 951. This makes it easy to perform proper treatment.

It is possible to estimate a therapeutic procedure effect by monitoring a temporal change in light intensity detected by the optical mammography apparatus 91.

In the first to fifth embodiments, when control is performed to perform, on the basis of plural kinds of living organism information detected by the plural medical apparatuses, a predetermined operation such as treatment for a therapeutic procedure (treatment of radiation of light or treatment of heat generation for killing a lesion tissue) or treatment related to the treatment (treatment for sampling a tissue or treatment for attaching a label to make it easy to sample a tissue), the plural kinds of living organism information may be used at different times or may be used at the same time.

The present invention is not limited to the case in which a gamma ray as radiation radiated from a drug accumulation region accumulated in a lesion tissue is detected. Radiation such as a beta ray or an alpha ray may be detected or fluorescent light may be detected as explained above.

An embodiment configured by, for example, partially combining the embodiments and the like also belong to the present invention.

With the medical systems according to the embodiments, it is possible to efficiently perform treatment or a predetermined operation related to the treatment for a living organism using the plural medical apparatuses having different functions for the living organism.

The present invention is not limited to the embodiments. Various alterations, modifications, and the like are possible in a range in which the gist of the present invention is not changed.

The present application is filed claiming the priority of Japanese Patent Application No. 2009-190288 filed in Japan on August 19, 2009, and the above described disclosure is incorporated by reference in the present description and claims.

**Claims**

1. A medical system comprising:

   a first medical apparatus including a first detecting unit for acquiring first living organism information from a living organism;
   a second medical apparatus including a second detecting unit for acquiring second living organism information from a living organism and an operation unit that performs a predetermined operation on the basis of the first living organism information received from the first medical apparatus; and
   a control unit provided in the second medical apparatus, the control unit controlling an operation of the operation unit on the basis of the second living organism information.

2. The medical system according to claim 1, wherein the first detecting unit is arranged outside the living organism, and the first living organism information is generated from detected information of the first detecting unit.

3. The medical system according to claim 1, wherein the first detecting unit is arranged inside the living organism, and the first living organism information is generated from detected information of the first detecting unit.

4. The medical system according to claim 2, wherein the second detecting unit and the operation unit are arranged outside the living organism, the second living organism information is generated from detected information of the second detecting unit, and the operation unit performs the predetermined operation from the outside of the living organism.

5. The medical system according to claim 2, wherein the second detecting unit and the operation unit are arranged inside the living organism, the second living organism information is generated from detected information of the second detecting unit, and the operation unit performs the predetermined operation from the inside of the living organism.

6. The medical system according to claim 3, wherein the second detecting unit and the operation unit are arranged inside the living organism, the second living organism information is generated from detected information of the second detecting unit, and the operation unit performs the predetermined operation from inside of the living organism.

**7.** The medical system according to claim 5, wherein the control unit includes a determining unit that performs, on the basis of the first living organism information and the second living organism information, determination whether a condition for causing the operation unit to perform the predetermined operation is satisfied, and the control unit causes the operation unit to perform the predetermined operation when a determination result indicates that the condition is satisfied.

**8.** The medical system according to claim 5, wherein the first detecting unit and the second detecting unit detect radiation radiated from a drug accumulation region of a specific drug provided with a characteristic of specifically combining with a lesion tissue in the living organism.

**9.** The medical system according to claim 7, wherein the predetermined operation is radiation of light or output of a radio wave for performing a therapeutic procedure for the lesion region in the drug accumulation region, treatment for sampling a tissue near the drug accumulation region, or an operation for attaching a label near the drug accumulation region.

**10.** The medical system according to claim 8 or 9, wherein the first or second medical apparatus is inserted into a living organism and is a capsule medical apparatus having a capsule shape.

**11.** The medical system according to claim 8, wherein
the second medical apparatus is inserted into a living organism and is a capsule medical apparatus having a capsule shape,
the first living organism information is position information on the drug accumulation region, and
the second living organism information is intensity information of the radiation.

**12.** The medical system according to claim 11, wherein the control unit includes a determining unit that performs, on the basis of the first living organism information and the second living organism information, determination whether a condition for causing the operation unit to perform the predetermined operation is satisfied, and the control unit causes the operation unit to perform the predetermined operation when a determination result indicates that the condition is satisfied.

**13.** The medical system according to claim 12, wherein the predetermined operation is radiation of light or output of a radio wave for performing a therapeutic procedure for the lesion region in the drug accumulation region, treatment for sampling a tissue near the drug accumulation region, or an operation for attaching a label near the drug accumulation region.

**14.** The medical system according to claim 1, wherein the second medical apparatus is an ultrasonic apparatus that radiates ultrasound.

**15.** A medical control method comprising:

a first step of detecting, with a first medical apparatus, a radiation emitted from a specific drug that specifically combines with a lesion tissue in a living organism and calculating a position and accumulation density of a drug accumulation region where the specific drug accumulates;
a second step of calculating, using information acquired by the first step, an estimation value of intensity of the radiation detected by a second medical apparatus at a predetermined distance from the drug accumulation region;
a third step of detecting radiation from the drug accumulation region with a second medical apparatus inserted into the living organism;
a fourth step of determining whether an intensity of radiation detected by the second medical apparatus exceeds the estimation value; and
a fifth step of performing control for applying, when a determination result indicates that an intensity of radiation detected by the second medical apparatus exceeds the estimation value, treatment for a therapeutic procedure to the drug accumulation region with the second medical apparatus.

**16.** The medical control method according to claim 15, wherein the second medical apparatus is inserted into a living organism and is a capsule medical apparatus having a capsule shape.

**17.** The medical control method according to claim 16, wherein the treatment for the therapeutic procedure is radiation of light or output of a radio wave for performing a therapeutic procedure for the lesion region in the drug accumulation region, treatment for sampling a tissue near the drug accumulation region, or an operation for attaching a label near the drug accumulation region.

# FIG.1

# FIG.2

# FIG.3

# FIG.4

# FIG.5

# FIG.6

START

S1 — DOSAGE OF MOLECULAR TARGET DRUG

S2 — IMAGE PICKUP (EXAMINATION) BY PET-CT APPARATUS

S3 — GENERATION AND DISPLAY OF PET-CT IMAGE AND CALCULATION OF POSITION AND ACCUMULATION DENSITY OF DRUG ACCUMULATION REGION

S4 — TRANSMISSION OF INFORMATION CONCERNING POSITION AND ACCUMULATION DENSITY

S5 — CALCULATION OF ESTIMATION VALUE Ies AT PREDETERMINED DISTANCE Ls

S6 — Ls IS PRESENT IN MOVING PASSAGE?  NO

YES

S7 — SWALLOW OF CAPSULE MEDICAL APPARATUS

S8 — TRANSMIT PICKED-UP IMAGE AND DETECTION SIGNAL OF GAMMA RAY BY RADIO

S9 — DISPLAY OF IMAGE AND INTENSITY Ide OF DETECTION SIGNAL

S10 — Ide > Ies ?  NO

YES

S11 — TRANSMIT CONTROL SIGNAL BY RADIO

S12 — LIGHT EMISSION OF LIGHT EMITTING UNIT

S13 — PERFORM TREATMENT OPERATION OF THERAPEUTIC TREATMENT ACCORDING TO GENERATION OF ACTIVE OXYGEN

S14 — STOP OF LIGHT EMISSION AND STOP OF IMAGE PICKUP

END

# FIG.7

# FIG.8

# FIG.9

# FIG.10

# FIG.11

# FIG.12

# FIG.13

MAIN BODY

TO CONTROL UNIT 97

# FIG.14

FOCUSED
ULTRASOUND

CONTROL UNIT

<div align="center">

**INTERNATIONAL SEARCH REPORT**

</div>

| International application No. |
| --- |
| PCT/JP2010/058000 |

**A.  CLASSIFICATION OF SUBJECT MATTER**
*A61N5/06*(2006.01)i, *A61B1/00*(2006.01)i, *A61B6/03*(2006.01)i, *G01T1/161*(2006.01)i

According to International Patent Classification (IPC) or to both national classification and IPC

**B.  FIELDS SEARCHED**

Minimum documentation searched (classification system followed by classification symbols)
A61N5/06, A61B1/00, A61B6/03, G01T1/161

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2005-334331 A  (Olympus Corp.),<br>08 December 2005 (08.12.2005),<br>entire text; fig. 1 to 30<br>& US 2005/0148847 A1    & EP 1669052 A1<br>& WO 2005/030114 A1 | 1-3,5-7<br>4,8-17 |
| Y | JP 2-126848 A  (Hitachi, Ltd.),<br>15 May 1990 (15.05.1990),<br>entire text; fig. 1 to 16<br>& US 5158071 A         & EP 351610 A2 | 4,14 |
| Y | JP 58-10067 A  (Olympus Optical Co., Ltd.),<br>20 January 1983 (20.01.1983),<br>entire text; fig. 1 to 4<br>(Family: none) | 8-13,15-17 |

☐  Further documents are listed in the continuation of Box C.       ☐  See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    28 May, 2010 (28.05.10) | Date of mailing of the international search report<br>    08 June, 2010 (08.06.10) |
| --- | --- |
| Name and mailing address of the ISA/<br>    Japanese Patent Office | Authorized officer |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5285098 A
- JP 2006304860 A
- JP 2009190288 A